# FASCICULE DE BREVET EUROPEEN

(11) **EP 3 631 421 B1**
(45) Date de publication et mention de la délivrance du brevet: **10.04.2024**
(21) Numéro de dépôt: 18726844.6
(22) Date de dépôt: 01.06.2018
(51) Int. Cl.: G01N 21/41, G01N 21/47, G01N 21/55, G01N 21/77

(54) **PROCEDE DE DETECTION OPTIQUE**
VERFAHREN ZUR OPTISCHEN DETEKTION
OPTICAL DETECTION METHOD

(30) Priorité: 02.06.2017 FR 1754930
(43) Date de publication de la demande: 08.04.2020
(73) Titulaire: Université de Lille, 59800 Lille (FR); Centre National de la Recherche Scientifique, 75794 Paris Cedex 16 (FR)
(72) Inventeur: VLANDAS, Alexis, 59650 Villeneuve d'Ascq (FR); WENGLER, Julien, 75005 Paris (FR); LAMANT, Sébastien, 59800 Lille (FR); SENEZ, Vincent, 59780 Baisieux (FR)
(74) Mandataire: Cabinet Nony
(86) Numéro de dépôt international: PCT/EP2018/064499
(87) Numéro de publication internationale: WO 2018/220191

(56) Documents cités:
- WO-A1-03/062334
- WO-A1-2012/004536
- JP-A- 2010 265 450
- US-A1- 2006 226 008
- US-A1- 2017 038 380
- HUIYAN LI ET AL: "Hydrogel droplet microarrays with trapped antibody-functionalized beads for multiplexed protein analysis", LAB ON A CHIP, vol. 11, no. 3, 1 janvier 2011 (2011-01-01), pages 528-534, XP055485674, ISSN: 1473-0197, DOI: 10.1039/C0LC00291G
- CHRISTIANE L. SALGADO ET AL: "Combinatorial cell-3D biomaterials cytocompatibility screening for tissue engineering using bioinspired superhydrophobic substrates", INTEGRATIVE BIOLOGY, vol. 4, no. 3, 1 janvier 2012 (2012-01-01) , page 318, XP055485666, Cambridge ISSN: 1757-9694, DOI: 10.1039/c2ib00170e
- SHOUICHI SAKAKIHARA ET AL: "A single-molecule enzymatic assay in a directly accessible femtoliter droplet array", LAB ON A CHIP, vol. 10, no. 24, 1 janvier 2010 (2010-01-01), page 3355, XP055085580, ISSN: 1473-0197, DOI: 10.1039/c0lc00062k
- Shouichi Sakakihara ET AL: "A single-molecule enzymatic assay in a directly accessible femtoliter droplet array", Lab on a Chip, vol. 10, no. 24, 1 January 2010 (2010-01-01), page 3355, XP055085580, ISSN: 1473-0197, DOI: 10.1039/c0lc00062k

## Description

La présente invention concerne la détection de la sensibilité d'un ou plusieurs polymères à un composé, en particulier celle d'un ou plusieurs biopolymères à une enzyme.

Dans la suite du document, on appelle 'polymère' selon l'invention un polymère naturel ou synthétique, homopolymère ou hétéropolymère, dont la structure peut être cristalline, amorphe ou hybride (amorphe et cristalline), ou un mélange de 'polymères' définis comme ci-dessus.

Le polymère selon l'invention (unique ou mélange) peut être complémenté par un agent chimique créant des liaisons covalentes entre les chaînes du polymère (ou du mélange de polymères). Cet agent chimique peut être une résine thermodurcissable, fonction des chaînes à lier. On appelle 'résine' un corps chimique ayant la capacité de former des liaisons covalentes avec le 'polymère' par un apport d'énergie de type thermique, photonique ou chimique.

Par 'composé' on entend un agent chimique ou biologique ayant la capacité de modifier chimiquement ou physiquement l'indice de réfraction et/ou la géométrie du dépôt de 'polymère'. Dans de nombreux domaines techniques et industriels, l'identification et l'étude d'enzymes ayant une activité de dégradation envers des polymères naturels ou synthétiques constituent un enjeu majeur.

Par exemple, certaines enzymes de dégradation, par exemple hydrolytiques constituent un outil essentiel pour générer des biocarburants à partir de la biomasse riche en lignocelluloses.

Plus précisément, la paroi végétale est une structure complexe formée de polymères enchevêtrés (cellulose, hémicelluloses, lignines, pectines, etc.) ; les enzymes de dégradation sont par exemple employées pour rompre les chaînes cellulosiques et hémicellulosiques en des sucres destinés à être fermentés afin de créer du bioéthanol, entre autres applications.

A cet effet, il est intéressant d'employer des dispositifs techniques qui permettent la détection, de manière rapide, simple et fiable, de l'activité de dégradation d'une enzyme, ou d'une combinaison d'enzymes, envers un polymère ou une combinaison de polymères.

Il est en particulier courant de mettre en oeuvre des dispositifs colorimétriques dédiés, qui utilisent des réactions chimiques pour révéler l'activité enzymatique. Ces dispositifs colorimétriques emploient pour cela des colorants ou des indicateurs chimiques de détection.

La plupart des tests enzymatiques qui existent permettent d'étudier une enzyme ou un type de microorganisme exprimant une enzyme sous le format de plaque multi-puits dans les puits de laquelle on vient ajouter un substrat (par exemple un polymère) qui, sous l'action de l'enzyme, se transforme en un produit fluorescent ou coloré. Ce format nécessite de produire des substrats modifiés (ajout d'une molécule fluorescente ou d'un composé chimique coloré). Il est aussi possible de révéler la présence d'un produit réactionnel par ajout d'un réactif chromogénique. Dans ces deux cas, les volumes de matériel sont de quelques microlitres au minimum. Il demande des outils robotiques évolués et coûteux, et des capteurs de photons extrêmement sensibles.

Les dispositifs colorimétriques, pour la détection de l'activité hydrolytique envers un ou plusieurs polymères, sont ainsi souvent complexes à utiliser, nécessitent de nombreuses manipulations et réactifs. Lorsqu'aucun substrat ou réactif chromogénique n'est utilisé, il est nécessaire d'employer des outils analytiques comme la spectrométrie de masse, la chromatographie, la spectrométrie infra-rouge ou encore la résonance magnétique nucléaire. Malgré les nombreux avantages de ces outils (sensibilité, spécificité de détection de l'acte catalytique), ils nécessitent des équipements coûteux et demandent une expertise particulière pour leur utilisation.

D'autres dispositifs utilisent l'interférométrie pour détecter l'activité de dégradation d'un polymère.

Ces dispositifs comportent pour cela au moins un mince film transparent de polymère rapporté sur un support réfléchissant (classiquement du silicium). L'épaisseur de ce film transparent est comprise dans une gamme de valeurs permettant l'apparition d'une couleur particulière due à un phénomène optique d'interférences, du fait qu'une partie du rayon incident est réfléchie à l'interface air/film, tandis qu'une autre partie du rayon incident est réfractée puis réfléchie à l'interface film / support. L'interférence des rayons réfléchis et des rayons réfractés crée une figure d'interférences. La couleur résultante correspond à la longueur d'onde où le phénomène d'interférences constructives est maximal.

Un dispositif de ce type est décrit dans la demande FR 2 962 545, étant prévu en particulier pour détecter l'activité hydrolytique envers un polymère, typiquement un polysaccharide, constituant principalement le matériau du film transparent mince déposé sur le support réfléchissant.

Les études menées ont montré que, pour un temps d'incubation de quelques dizaines de minutes, cette méthode possède un seuil de sensibilité de 125 à 1 000 fois plus faible que les méthodes classiquement employées dans l'identification d'enzymes.

Cette méthode présente toutefois certaines limitations.

Elle provoque en effet une importante perte de matière lors de la fabrication du film mince puisqu'une grande partie de la surface du support doit être revêtue de polymère, et ce de manière continue, pour permettre la manifestation d'interférences lumineuses. En utilisant la technologie d'enduction centrifuge, la perte de matière est considérable (plus de 90%), ce qui présente un inconvénient majeur en vue d'une industrialisation, ou bien lorsque le polymère à tester n'est disponible qu'en très faible quantité.

L'étalement d'un unique polymère ou d'un unique mélange de polymères par support représente également une limite de cette méthode.

Les articles de Huiyan Li et al. [1] et de Christiane L. Salgado et al. [2] décrivent tous deux des dépôts d'hydrogel à base d'alginate, ces dépôts étant immergés dans une solution de CaCl₂ pour permettre la gélification de l'alginate et donc la formation d'hydrogel.

La demande de brevet US 2017/0038380 A1 décrit un procédé d'analyse de la cinétique d'interaction protéique comportant le dépôt de gouttelettes d'analyte sur des régions prédéfinies de la surface d'un support comportant des molécules échantillons. Le procédé comporte la détection par résonnance plasmonique de surface dans laquelle on éclaire la face du support opposée à la surface comportant les molécules échantillons.

L'article de Shouichi Sakakihara et al. [3] décrit un réseau de gouttelettes dans lequel les gouttelettes sont fixées sur le substrat et sont directement accessibles de l'extérieur.

Il existe donc un besoin pour un nouveau procédé de détection d'activité enzymatique remédiant à tout ou partie des inconvénients ci-dessus, et en particulier ne nécessitant pas une grande quantité de polymère, et/ou permettant de tester aisément plusieurs polymères et/ou plusieurs enzymes avec un même dispositif.

L'invention vise également à disposer, si cela est souhaité, d'un procédé permettant de mesurer précisément et quantitativement l'hydrolyse d'un polymère par une enzyme, par exemple en suivant la cinétique d'hydrolyse. L'invention est définie dans les revendications.

### Procédé de détection

Selon un premier objet, la présente description, mais ne faisant pas partie de l'invention, concerne un procédé de détection de la sensibilité d'un polymère ou d'un mélange de polymères à un composé, comportant les étapes consistant à :
- exposer au moins un micro-dépôt, de préférence de forme lenticulaire, comportant le polymère ou le mélange de polymères, au composé,
- détecter un changement de la distribution spatiale de l'intensité de la lumière réfléchie ou transmise par ce micro-dépôt, lié à une variation des dimensions et/ou de l'indice de réfraction de ce micro-dépôt, sous l'effet d'une interaction entre le polymère ou le mélange de polymères et le composé.

La présente invention concerne aussi un procédé de détection de la sensibilité d'un ou plusieurs polymères et/ou d'un ou plusieurs mélanges de polymères à un composé, comportant les étapes consistant à :
- exposer au moins un micro-dépôt solide de forme lenticulaire, comportant le ou les polymères et/ou le ou les mélanges de polymères, au composé,
- détecter en éclairant la surface de ce micro-dépôt, un changement de la distribution spatiale de l'intensité de la lumière réfléchie ou transmise par ce micro-dépôt, lié à une variation des dimensions et/ou de l'indice de réfraction de ce micro-dépôt, sous l'effet d'une interaction entre le ou les polymères et/ou le ou les mélanges de polymères et le composé.

Les micro-dépôts selon l'invention peuvent être monocouches, c'est-à-dire comporter une seule couche et/ou être multicouches, c'est-à-dire comporter plusieurs couches superposées.

Chaque couche peut comporter le polymère ou le mélange de polymères.

Dans le cas où le micro-dépôt est monocouche, ce dernier peut se présenter sous la forme d'un plot unique comportant le polymère ou le mélange de polymères. La monocouche est disposée sur un support, de préférence au contact de ce dernier. De préférence, cette monocouche est de forme lenticulaire.

Dans le cas où le micro-dépôt est multicouches, ce dernier peut présenter deux couches, notamment avec une architecture coeur-coquille (« core-shell » en anglais). Le micro-dépôt peut alors présenter une première couche correspondant à un coeur, et une deuxième couche correspond à une coquille.

La première couche peut être disposée sur le support, de préférence au contact de ce dernier, et peut être de forme lenticulaire.

La deuxième couche peut recouvrir au moins partiellement, de préférence intégralement, la première couche et peut présenter une forme complémentaire de la première couche.

Les première et deuxième couches peuvent chacune présenter une face en regard du support dite face intérieure et une face opposée à la face en regard du support dite face extérieure.

De préférence, la face intérieure de la première couche est au contact du support.

De préférence, la face extérieure de la première couche est convexe et au contact de la face intérieure de la deuxième couche.

Le micro-dépôt comportant deux couches avec une architecture coeur-coquille peut présenter un coeur comprenant par exemple un seul premier polymère et une coquille comprenant par exemple un seul deuxième polymère différent du premier, ou un coeur comprenant plusieurs polymères et une coquille comprenant plusieurs polymères, ou un coeur comprenant un seul polymère et une coquille comprenant plusieurs polymères, ou encore un coeur comprenant plusieurs polymères et une coquille comprenant un seul polymère.Le type de micro-dépôts choisi est adapté à l'application visée.

L'étape d'exposition du ou des micro-dépôts au composé peut comprendre une période d'incubation à température ambiante, qui peut durer par exemple de 1 à 240 minutes. Cette étape peut être suivie d'une étape de lavage et d'une étape de séchage.

Lorsque l'interaction entre le ou les polymères et le composé est une dégradation du ou des polymères par le composé, le micro-dépôt contenant le ou les polymères va diminuer de volume. Si le ou les polymères ne sont pas sensibles au composé mis en présence, le micro-dépôt contenant le ou les polymères va maintenir son volume et sa forme.

De préférence, lorsque l'interaction entre le polymère et le composé est une dégradation du polymère par le composé, le micro-dépôt contenant le polymère va diminuer de volume. Si le polymère n'est pas sensible au composé mis en présence, le micro-dépôt contenant le polymère va maintenir son volume et sa forme.

La diminution de volume des micro-dépôts provoque, à l'échelle macroscopique, une différence d'aspect par rapport à ceux conservant leur volume. Cette différence de contraste peut être observable à l'oeil nu.

Ainsi, selon un exemple de mise en oeuvre de l'invention, une comparaison de l'aspect d'un ensemble de micro-dépôts exposé au composé et d'un ensemble de micro-dépôts non exposé au composé est effectuée, en vue de détecter un changement d'aspect. Cette comparaison peut s'effectuer sans agrandissement, en lumière réfléchie.

Selon un mode de réalisation, l'observation de l'apparition du contraste s'effectue à l'aide d'un ou plusieurs instruments de mesure appropriés, notamment à l'aide d'une caméra et de préférence d'un programme de traitement d'image. Ce programme peut notamment détecter automatiquement une variation d'aspect et l'apparition d'un contraste, voire le quantifier.

L'invention permet aussi de mesurer la dégradation des micro-dépôts en mesurant l'altération de la réponse optique de champ lointain des micro-dépôts. Cette méthode offre une très bonne sensibilité due à la taille micrométrique des dépôts et permet une lecture quantitative facile et peu coûteuse à mettre en oeuvre, grâce à l'observation simple à l'aide d'une caméra ou de tout autre capteur d'intensité.

Dans un tel procédé selon l'invention, plusieurs micro-dépôts peuvent être exposés au composé et la détection du changement de la distribution spatiale de l'intensité lumineuse s'effectue par l'observation de ces micro-dépôts.

La détection du changement de la distribution spatiale peut notamment être observée avec ou sans grossissement.

Le ou les micro-dépôts peuvent être éclairés par une source ponctuelle ou linéaire, mieux linéaire.

L'observation de la distribution spatiale de l'intensité de la lumière peut s'effectuer au voisinage de la réflexion spéculaire, notamment dans une plage 0°-2,5° à partir de la réflexion spéculaire.

L'observation du changement de la distribution spatiale peut s'effectuer avec un capteur matriciel.

Les micro-dépôts peuvent être disposés selon tout type d'arrangement, périodique ou non. Les micro-dépôts peuvent notamment être disposés sous forme de réseau ou de matrice.

Selon un mode de réalisation, les micro-dépôts sont disposés en un réseau périodique et éclairés avec une lumière cohérente, notamment de type LASER. L'observation de la distribution spatiale de la lumière dans une figure de diffraction produite par le réseau permet de mesurer la dégradation des micro-dépôts.

Une information concernant la variation du volume du micro-dépôt peut être générée à partir du changement observé, par comparaison à des données de référence, celles-ci pouvant être expérimentales ou obtenues par modélisation à partir de la forme des dépôts.

Le composé peut être une enzyme, l'interaction entre le composé et le ou les polymères, de préférence entre le composé et le polymère, étant de préférence une dégradation du ou des polymères, de préférence du polymère, par le composé.

L'interaction ci-dessus peut s'accompagner d'une variation d'une figure d'interférences au niveau de chaque micro-dépôt.

La variation de la figure d'interférences offre lorsqu'elle existe un moyen de mesure plus fin de l'interaction.

La détection d'une variation d'une figure d'interférences, lorsqu'elle existe, peut s'effectuer par interférométrie. De préférence, cette détection par interférométrie s'effectue après grossissement de la figure d'interférences observée sur chaque micro-dépôt. La détection par interférométrie peut s'accompagner avantageusement de la reconstruction du volume du micro-dépôt à partir de la localisation des franges d'interférences.

L'épaisseur des micro-dépôts est de préférence comprise entre 100 nm et 5000 nm, mieux entre 100 et 1500 nm, l'épaisseur de chaque micro-dépôt passant avantageusement par un seul maximum.

Ainsi, selon un mode de réalisation, chaque micro-dépôt a une surface extérieure convexe.

La plus grande dimension des micro-dépôts est de préférence comprise entre 10 µm et 100 µm.

De préférence, les micro-dépôts ont un contour de forme sensiblement circulaire.

Les micro-dépôts, d'échelle micrométrique, sont de préférence déposés de façon à former des macro-motifs discernables les uns des autres à l'oeil nu, d'échelle millimétrique. Ces macro-motifs sont de préférence distants et disjoints les uns des autres.

Les macro-motifs peuvent être disposés comme les puits d'une grille multi-puits pour dépôt robotisé, par exemple une grille à 96 puits.

Cela peut faciliter l'emploi d'outils robotiques pour déposer un ou plusieurs composés à tester sur les macro-motifs et ensuite l'analyse des variations d'aspect éventuelles au niveau de chaque macro-motif, de façon analogue à l'observation d'une réaction au sein de chaque puits.

Selon un mode de réalisation préféré, les micro-dépôts sont disposés sous la forme de points de trame (classique ou stochastique), l'espacement entre les points de trame étant de préférence suffisamment faible pour que le revêtement formé par les micro-dépôts apparaisse, au sein d'un macromotif, continu à l'oeil nu à 25 cm de distance. La trame peut être régulière, ainsi que la taille des micro-dépôts. Autrement dit, chaque micro-dépôt peut ne pas être discernable à l'oeil nu, tandis que les macro-motifs le sont.

Il existe de nombreuses techniques qui permettent de réaliser un tel arrangement de micro-dépôts : la photolithographie (Rao et al, 2014), le microcontact (Voskuhl, et al, 2014) et l'impression jet d'encre (Komuro, et al, 2013).

Selon un mode de réalisation préféré, les micro-dépôts sont obtenus par impression par jet d'encre.

Les avantages de l'impression jet d'encre sont la faible valeur des volumes éjectés (de l'ordre du picolitre, modulable selon les propriétés du liquide ou les paramètres d'éjection, permettant une grande densité de micro-dépôts), l'absence de contact avec le support solide (supprime tout dommage du support et/ou de la tête d'impression), l'existence d'un réservoir d'encre externe à la tête d'impression, qui rend possible la réalisation de plusieurs milliers de macro-motifs de micro-dépôts avant épuisement de l'encre, ce qui améliore grandement la reproductibilité des dépôts, et une haute cadence d'impression, par exemple jusqu'à 500 gouttelettes à la seconde.

Le support sur lequel les micro-dépôts sont situés peut être solide ou flexible, plan ou non.

Le support est de préférence non poreux et lisse.

En variante, le support est nanostructuré pour aider au contrôle de la mouillabilité.

Selon un mode de réalisation, le support consiste en un matériau opaque ou transparent présentant une surface supérieure ayant des propriétés réfléchissantes.

Par « réfléchissante », on entend ici une réflexion optique totale ou partielle, de type spéculaire. La réflexion concerne de préférence la plage de longueurs d'ondes du domaine visible.

Le support consiste avantageusement en une galette de silicium (couramment dénommée « wafer »).

De préférence, les micro-dépôts sont situés sur un support hydrophobe. L'hydrophobie du support réduit le risque de coalescence de gouttelettes lors de l'impression, et facilite l'obtention d'une forme convexe vers l'extérieur.

Le support peut être rendu hydrophobe par toute technique connue de l'homme du métier.

Un traitement par des composés perfluorés, tels que des composés porteurs de groupes perfluorocarbonés, peut être mis en oeuvre. On peut citer en exemple le traitement par le gaz C₄F₈.

En variante, un traitement au perfluorodecyltrichlorosilane (FDTS) est mis en oeuvre.

Dans une autre variante, un traitement par voie humide est mis en oeuvre, par exemple par immersion du support pendant une durée de 2 à 18h dans une solution préparée selon les spécificités ci-après, puis séchage :

| | |
|---|---|
| Hexane | 14 mL |
| Dichlorométhane | 6 mL |
| Acide acétique | 0,1 mL |
| 3-methacryloxypropyltrimethoxysilane | 0,1 mL |

Le support peut présenter un revêtement hydrophobe de 100 nm d'épaisseur ou moins, sur lequel les micro-dépôts sont réalisés.

Le composé dont on étudie l'interaction avec le ou les polymères et/ou le ou les mélanges de polymères, de préférence avec le polymère ou le mélange de polymères, est typiquement une enzyme ou un ensemble d'enzymes, l'interaction entre le composé et le ou les polymères, de préférence entre le composé et le polymère, étant de préférence une dégradation enzymatique, notamment une hydrolyse enzymatique du ou des polymères, de préférence du polymère, par le composé.

Le ou les polymères peuvent être dégradables par une enzyme ou un ensemble d'enzymes.

A titre d'enzyme particulièrement adaptée, on peut citer les enzymes de type hydrolase.

Lors de l'étape d'exposition des micro-dépôts au composé, ce dernier est de préférence en solution, notamment en solution aqueuse.

Lorsqu'il s'agit d'une enzyme, celle-ci est typiquement en solution aqueuse, dans des conditions propices à une hydrolyse enzymatique.

Par « hydrolyse enzymatique », on désigne un mécanisme mis en oeuvre par une enzyme de type hydrolase, catalysant une réaction biochimique d'hydrolyse.

Parmi les enzymes de type hydrolase, on peut citer en particulier les enzymes ayant une activité hydrolytique envers les biopolymères.

Parmi ces enzymes aptes à hydrolyser des biopolymères, on peut citer en particulier les glycosides hydrolases, qui incluent les glycosidases.

Par « glycoside hydrolases », on englobe entre autres les xylanases, les cellulases, les chitinases, les pectinases, les mannases, et la Cellulyve (marque déposée).

Parmi les enzymes hydrolytiques de biopolymères, on peut citer par exemple les protéases, les estérases, les nucléases, les ligninases.

Le polymère ou le mélange de polymères étudié par le procédé de l'invention est de préférence présent dans les micro-dépôts selon une teneur comprise de 99,5% à 95% en poids par rapport au poids total de la couche desdits dépôts dans laquelle il est présent.

De préférence, le polymère étudié par le procédé de l'invention est de préférence présent dans les micro-dépôts selon une teneur comprise de 99,5% à 95% en poids par rapport au poids total desdits dépôts.

Le ou les polymères, de préférence le polymère, peut être choisi parmi les biopolymères.

De tels biopolymères peuvent se présenter sous une forme isolée (c'est-à-dire des chaînes de polymères distinctes), agrégée (c'est-à-dire des chaînes de polymères entremêlées) ou cristallisée (c'est-à-dire des chaînes de polymères organisées selon un motif répétitif ordonné).

Le ou les polymères peuvent être des bio-polymères, notamment choisi dans le groupe constitué par les oligosaccharides et polysaccharides tels que la cellulose, le xylane, la pectine, le chitosane, la chitine, le xyloglucane, le bétaglucan, et l'arabinoxylane ; les peptides et les protéines tels que l'albumine de sérum bovin ou humain et la gluténine ; les acides nucléiques tels que les acides désoxyribonucléiques et les acides ribonucléiques ; la cutine, la subérine et la lignine.

De préférence, le polymère est un bio-polymère, notamment choisi dans le groupe constitué par les oligosaccharides et polysaccharides tels que la cellulose, le xylane, la pectine, le chitosane, la chitine, le xyloglucane, et l'arabinoxylane ; les peptides et les protéines tels que l'albumine de sérum bovin ou humain et la gluténine ; les acides nucléiques tels que les acides désoxyribonucléiques et les acides ribonucléiques ; la cutine, la subérine et la lignine.

Le mélange de polymères peut comporter un bio-polymère, notamment choisi dans le groupe constitué par les oligosaccharides et polysaccharides tels que la cellulose, le xylane, la pectine, le chitosane, la chitine, le xyloglucane, le bétaglucan, et l'arabinoxylane ; les peptides et les protéines tels que l'albumine de sérum bovin ou humain et la gluténine ; les acides nucléiques tels que les acides désoxyribonucléiques et les acides ribonucléiques ; la cutine, la subérine et la lignine.

Le ou les polymères, de préférence le polymère, est avantageusement immobilisé sur le support.

Cette immobilisation peut être générée par des liaisons (i) de type covalentes ou « chimiques » (réticulation) et/ou (ii) de type non covalentes ou « physiques » (électrostatiques, hydrogènes, force de Van der Waals).

Dans le cas (i) ci-dessus, les micro-dépôts contiennent par exemple une résine présente selon une teneur comprise de 0,5% à 5% en poids par rapport au poids total des micro-dépôts, cette résine étant de préférence une résine thermodurcissable, par exemple mélamine-formaldéhyde.

Pour le choix et la mise en oeuvre d'une telle résine, on peut se référer aux documents suivants : Ducéré et al. Sensors and Actuators B: Chemical 2005, 106(1), 331-334, Wu et al. Séparation and Purification Technology 2009, 68(1), 97-104, et Schuler et al. Biomacromolecules 2001, 2(3), 921-926.

Par exemple, la réticulation par des résines mélamine-urée-formaldéhyde ou mélamine-formaldéhyde (désignées encore « MUF » ou « MF ») présente les avantages suivants :
- la réaction de réticulation peut avoir lieu avec de nombreuses fonctions chimiques (alcool, amine, phénol), permettant ainsi de réticuler un grand nombre de classes de biopolymères ;
- il existe des formulations de résine soluble dans l'eau;
- la réticulation est simple : le monomère est stable à température ambiante et il réagit lorsque le film est porté à 90°C en atmosphère sèche pendant une heure, ce traitement étant compatible avec la majorité des biopolymères.

Dans le mode de réalisation où le composé est une enzyme, le procédé selon l'invention permet d'analyser l'activité de dégradation enzymatique de polymères, ou mélanges de polymères, naturels, typiquement de biopolymères tels que des polysaccharides ou des lignines, ou de polymères synthétiques, dans un contexte où le nombre d'enzymes à tester est grand et le coût du test est réduit. L'invention permet de réaliser le test sur le terrain, à l'oeil nu, sans équipement particulier.

Lorsque l'interaction entre le polymère et le composé provoque une dégradation du polymère par le composé, le micro-dépôt contenant le polymère va diminuer de volume. Si le polymère n'est pas sensible au composé mis en présence, le micro-dépôt contenant le polymère va maintenir sensiblement son volume et sa forme.

La diminution de volume des micro-dépôts provoque, à l'échelle macroscopique, une différence d'aspect par rapport à ceux conservant sensiblement leur volume. Cette différence de contraste est observable à l'oeil nu.

Selon un exemple de mise en oeuvre de l'invention, une comparaison de l'aspect d'un ensemble de micro-dépôts exposé au composé et d'un ensemble de micro-dépôts non exposé au composé est effectuée, en vue de détecter un changement d'aspect. Cette comparaison peut s'effectuer sans agrandissement, en lumière réfléchie.

Selon un mode de réalisation, l'observation de l'apparition du contraste s'effectue à l'aide d'un ou plusieurs instruments de mesure appropriés, notamment à l'aide d'une caméra et de préférence d'un programme de traitement d'image. Ce programme peut notamment détecter automatiquement une variation d'aspect et l'apparition d'un contraste, voire le quantifier. la description, mais ne faisant pas partie de l'invention, a encore pour objet, selon un autre de ses aspects, un procédé de détection de la sensibilité d'un polymère à un composé, comportant les étapes consistant à :
- exposer un arrangement périodique-de micro-dépôts, notamment de forme lenticulaire, comportant le polymère, au composé,
- détecter en exposant cet arrangement périodique à une lumière adaptée, notamment cohérente, un changement de la distribution spatiale de l'intensité de la lumière diffractée par ce réseau, lié à une variation des dimensions et/ou de l'indice de réfraction des micro-dépôts de cet arrangement sous l'effet d'une interaction entre le polymère et le composé.

L'invention concerne aussi un procédé de détection de la sensibilité d'un ou plusieurs polymères à un composé, comportant les étapes consistant à :
- exposer un arrangement périodique-de micro-dépôts solides de forme lenticulaire, comportant le ou les polymères, au composé,
- détecter en exposant la surface cet arrangement périodique à une lumière adaptée, notamment cohérente, un changement de la distribution spatiale de l'intensité de la lumière diffractée par ce réseau, lié à une variation des dimensions et/ou de l'indice de réfraction des micro-dépôts de cet arrangement sous l'effet d'une interaction entre le ou les polymères et le composé.

L'invention permet 1) d'identifier le composé ou le mélange de composés qui par exemple dégrade le plus efficacement un polymère ou un mélange de polymères et 2) de classer un ensemble de composés ou de mélange de composés par rapport à leur cinétique par exemple de dégradation d'un polymère ou d'un mélange de polymères.

L'invention permet si on le souhaite de remonter à la perte de volume des dépôts solides induite par la dégradation du fait de la réaction enzymatique. Il permet de faire ceci soit de façon quantitative dans le contexte d'un laboratoire à l'aide d'un système optique, soit de façon qualitative sur le terrain à l'oeil nu ou potentiellement de façon quantitative à l'aide d'un instrument de lecture portable.

Des avantages du procédé selon des variantes préférées de mise en oeuvre de l'invention sont les suivants :
- possibilité d'étudier un composé unique ou un cocktail de composés,
- capacité à déposer un biopolymère/polymère ou un ensemble de polymères ou mélanges de polymères de compositions différentes sur un même support, au niveau d'un même motif ou d'un macro-motif à l'autre,
- détection de l'interaction composé-polymère sans utilisation de marqueur,
- niveau de sensibilité équivalent ou meilleur aux techniques biochimiques classiques utilisant un substrat fluorogène ou chromogène pour un temps d'incubation équivalent,
- possibilité de moduler la dynamique de détection en fonction du type d'échantillon testé (propriétés physico-chimiques du dépôt),
- intégration d'un double niveau d'analyse : a) macroscopique permettant de visualiser s'il y a hydrolyse ou pas à l'oeil nu, b) macroscopique à l'aide d'instrument compatible au système de détection avec les technologies embarquées sur les téléphones portables récents permettant d'extraire des données quantitative sur les activités des composés.

Dans le cadre du diagnostic fonctionnel d'écosystèmes microbiens ou de souches isolées, la capacité de fabriquer un test enzymatique à bas coût, jetable, et donnant rapidement une réponse est d'un intérêt certain. En effet, que ce soit pour des applications environnementales (performances de dégradation d'un polymère polluant), en santé humaine (dégradation du mucus intestinal par des commensales opportunistes, ou des pathogènes), ou en alimentation animale (performances de dégradation des fibres alimentaires par l'écosystème digestif ou par des cocktails d'enzymes exogènes rajoutés dans l'alimentation pour augmenter l'efficacité énergétique), le dosage semi-quantitatif des activités de dégradation de polymères est un pré-requis nécessaire à la caractérisation et à l'ingénierie des écosystèmes pour optimiser les services rendus (au niveau environnemental, ou pour la santé de l'hôte quand il s'agit d'écosystème humain ou animal).

L'utilisation actuelle d'enzymes dans certains secteurs industriels (textile, alimentation humaine et animale) demande au cours de la production de réaliser des tests pour vérifier la conformité du produit final, et le procédé selon l'invention trouve ainsi à s'appliquer également au contrôle qualité.

La description, mais ne faisant pas partie de l'invention, a encore pour objet un procédé de détection d'un changement d'état d'un polymère sous l'action d'un composé auquel le polymère est sensible, comportant les étapes consistant à :
- exposer au moins un micro-dépôt de forme lenticulaire, comportant le polymère, à des conditions provoquant un changement d'état au moins partiel,
- détecter un changement de la distribution spatiale de l'intensité de la lumière réfléchie ou transmise par ce micro-dépôt, lié à une variation des dimensions et/ou de l'indice de réfraction de ce micro-dépôt sous l'effet de ce changement d'état

L'invention concerne aussi un procédé de détection d'un changement d'état d'un ou plusieurs polymères sous l'action d'un composé auquel le ou les polymères sont sensibles, comportant les étapes consistant à :
- exposer au moins un micro-dépôt solide de forme lenticulaire, comportant le ou les polymères, à des conditions provoquant un changement d'état au moins partiel,
- détecter en éclairant la surface de ce micro-dépôt, un changement de la distribution spatiale de l'intensité de la lumière réfléchie ou transmise par ce micro-dépôt, lié à une variation des dimensions et/ou de l'indice de réfraction de ce micro-dépôt sous l'effet de ce changement d'état.

### Dispositif

L'invention concerne aussi un dispositif, particulièrement adapté pour la mise en oeuvre du procédé selon l'invention tel que défini plus haut, comportant :
- un support,
- une pluralité de micro-dépôts solides individualisés portés par le support, de forme lenticulaire, chaque micro-dépôt comportant au moins un polymère sensible à un composé.

Les caractéristiques de l'invention décrites ci-dessus en référence au procédé valent également pour le dispositif, et inversement.

Les micro-dépôts peuvent être disposés de façon suffisamment proche pour que l'effet d'interaction entre le ou les polymères et le composé, de préférence entre le polymère et le composé, soit visible à l'oeil nu sous forme d'un contraste d'intensité.

Le dispositif peut comporter un système optique comportant par exemple une source lumineuse éclairant à angle fixe la surface du dispositif et un capteur optique (caméra ou autre) collectant la lumière réfléchie par les micro-dépôts agissant comme des microlentilles.

### Ensemble

La description, mais ne faisant pas partie de l'invention, a encore pour objet un ensemble comportant un dispositif selon l'invention et un composé interagissant avec ledit polymère pour conduire à une variation des dimensions et/ou de l'indice de réfraction des micro-dépôts. Cette variation peut résulter d'une réaction de dégradation enzymatique entre le polymère et le composé.

L'invention concerne aussi un ensemble comportant un dispositif selon l'invention et un composé interagissant avec le ou les polymères pour conduire à une variation des dimensions et/ou de l'indice de réfraction des micro-dépôts. Cette variation peut résulter d'une réaction de dégradation enzymatique entre le ou les polymères et le composé.

### Procédé de fabrication du dispositif

L'invention concerne encore un procédé de fabrication d'un dispositif selon l'invention, comportant le dépôt par impression sur le support de points d'une encre contenant le ou les polymères pour former les polymères, un liant et un co-solvant organique choisi pour permettre la formation des micro-dépôts de forme convexe lors du séchage de l'encre sur le support.

La description, mais ne faisant pas partie de l'invention, concerne encore un procédé de fabrication d'un dispositif selon l'invention, comportant le dépôt par impression par jet d'encre sur le support de points d'une encre contenant le ou les polymères pour former les micro-dépôts.

La description, mais ne faisant pas partie de l'invention, concerne aussi un procédé de fabrication d'un dispositif selon l'invention, comportant le dépôt par impression sur le support de points d'une encre contenant ledit polymère pour former les micro-dépôts.

De préférence, la technique d'impression est de type jet d'encre.

Selon un mode de réalisation préféré, ce procédé de fabrication comprend une étape de réticulation de l'encre de manière à convertir les points d'encre en des micro-dépôts solides individualisés.

Le procédé peut comporter la modification de la mouillabilité du support préalablement à l'impression des micro-dépôts, afin d'ajuster l'épaisseur des micro-dépôts, notamment d'obtenir une forme convexe pour ceux-ci et de jouer sur l'homogénéité de l'épaisseur.

Selon une variante du procédé de fabrication du dispositif, le dépôt par impression sur le support s'effectue en plusieurs passages successifs superposés spatialement, de manière à obtenir des micro-dépôts multicouches, chaque passage permettant de déposer une couche d'encre.

De préférence, le nombre de passage est égal à deux. Cela permet de former sur le support des micro-dépôts comportant deux couches superposées, notamment avec une architecture coeur-coquille (« core-shell » en anglais). Dans ce cas, le micro-dépôt présente une première couche correspondant au coeur, et une deuxième couche correspond à la coquille.

La première couche peut être disposée sur le support, de préférence au contact de ce dernier, et peut être de forme lenticulaire.

La deuxième couche peut recouvrir au moins partiellement, de préférence intégralement, la première couche et peut présenter une forme complémentaire de la première couche.

Dans une première étape, on dépose lors d'un premier passage la première couche sur le support, de préférence au contact de ce dernier.

Dans une deuxième étape, on dépose lors d'un deuxième passage la deuxième couche de manière à ce que cette dernière vienne recouvrir au moins partiellement, de préférence intégralement, la première couche.

Le dépôt de la deuxième couche est de préférence effectué lorsque la première couche est réticulée.

Il est ainsi possible, en utilisant une encre différente dans la première et deuxième couche, d'obtenir des micro-dépôts constitués de couches de natures différentes, par exemple comportant chacune un polymère distinct, ou bien encore des ratios de polymères mélangés différents.

L'épaisseur des micro-dépôts multicouches est de préférence comprise entre 100 nm et 1 500 nm, mieux entre 100 et 1000 nm, l'épaisseur de chaque micro-dépôt multicouche passant avantageusement par un seul maximum.

La plus grande dimension des micro-dépôts multicouches est de préférence comprise entre 10 µm et 100 µm.

L'épaisseur de la première couche du micro-dépôt multicouche est de préférence comprise entre 30 nm et 1300 nm, mieux entre 70 nm et 800 nm.

La plus grande dimension de la première couche du micro-dépôt multicouche est de préférence comprise entre 5 µm et 95 µm.

### Encre aqueuse

La description, mais ne faisant pas partie de l'invention, a encore pour objet une encre, de préférence aqueuse, particulièrement adaptée à la mise en oeuvre du procédé de fabrication d'un dispositif selon l'invention, comportant :
- le ou les polymères, de préférence le polymère,
- un liant, de préférence une résine, notamment mélamine formaldéhyde, et
- un co-solvant organique choisi pour permettre la formation d'un micro-dépôt de forme convexe lors du séchage de l'encre, de préférence du diméthylsulfoxyde (DMSO).

La description, mais ne faisant pas partie de l'invention, concerne aussi une encre, de préférence aqueuse, particulièrement adaptée à la mise en oeuvre du procédé de fabrication d'un dispositif selon l'invention, comportant :
- Le ou les polymères, notamment dégradables par une enzyme, de préférence choisi parmi les oligosaccharides et polysaccharides tels que la cellulose, le xylane, la pectine, le chitosane, la chitine, le xyloglucane, le bétaglucan, et l'arabinoxylane ; les peptides et les protéines tels que l'albumine de sérum bovin ou humain et la gluténine ; les acides nucléiques tels que les acides désoxyribonucléiques et les acides ribonucléiques ; la cutine, la subérine et la lignine,
- un liant, de préférence une résine, notamment mélamine formaldéhyde, et
- un co-solvant organique choisi pour permettre la formation d'un micro-dépôt de forme convexe lors du séchage de l'encre, de préférence du DMSO,
l'encre étant de préférence conditionnée dans une cartouche pour imprimante à jet d'encre.

De préférence, la teneur en polymère dans l'encre est comprise de 1 mg/mL à 10 mg/mL par rapport au volume de l'encre.

Dans le cas d'un mélange de polymères, les proportions de chacun des polymères dans le mélange sont choisies en fonction de l'application visée.

De préférence, la teneur en résine dans l'encre est comprise 0,01 mg/mL et 0,1 mg/mL par rapport au volume de l'encre.

Le ratio polymère(s)/résine peut être compris entre 100 et 300.

Le ratio polymère(s)/résine est de préférence égal à 200.

La viscosité de l'encre à 20°C est de préférence comprise entre 5 mPa.s et 20 mPa.s, préférentiellement égale à 15 mPa.s +/- 3 mPa.s., notamment à 17 mPa.S.

La tension de surface est par exemple de 62 mN.m⁻¹, étant de préférence inférieure ou égale à 70 mN.m⁻¹.

L'encre est de préférence conditionnée dans une cartouche pour imprimante à jet d'encre.

L'encre peut comprendre au moins un agent filmogène, ce dernier permettant la formation aisée du micro-dépôt.

Ledit agent filmogène est choisi par exemple parmi : les polyallylamine, les polyacrylamide, les polyvinylpyrrolidone, et leurs copolymères.

Préférentiellement, ledit agent filmogène est le chlorohydrate de Poly(allylamine) PAH.

La teneur en agent filmogène dans l'encre est préférentiellement comprise entre 0% et 10% en volume.

L'encre peut comprendre au moins un agent catalytique, ce dernier permettant de faciliter la réticulation du micro-dépôt.

Ledit agent catalytique peut être choisi en fonction de son influence sur la température de recuit nécessaire à la formation du micro-dépôt.

Ledit agent catalytique est choisi par exemple parmi les acides.

Préférentiellement, ledit agent catalytique est l'acide chlorhydrique.

La teneur en agent catalytique dans l'encre est préférentiellement comprise entre 0% et 10% en volume.

L'encre est de préférence dépourvue de pigments et de colorants.

La présence du co-solvant organique est utile pour éviter l'effet "tache de café" lors du séchage de l'encre, conduisant à la formation d'un dépôt d'épaisseur moins homogène sur le support. D'autres solvants que le DMSO sont utilisables, par exemple l'éthylène glycol, ou le *N,N*-diméthylformamide (DMF) , entre autres.

L'invention pourra être mieux comprise à la lecture de la description détaillée qui va suivre, d'exemples de mise en oeuvre non limitatifs de celle-ci, ainsi qu'à l'examen du dessin annexé, sur lequel :
- la figure 1 représente en vue de dessus un exemple de dispositif selon l'invention,
- la figure 2 représente de façon schématique à échelle agrandie un macro-motif,
- la figure 3 représente en coupe des micro-dépôts,
- la figure 4 illustre l'observation d'un micro-dépôt en réflexion, avant dégradation,
- la figure 5 illustre l'effet de la dégradation sur la distance focale,
- les figures 6A à 6D représentent des images obtenues par observation au microscope optique des micro lentilles,
- la figure 7 est un résultat de simulation faisant apparaître le lien entre la dégradation et l'intensité à angle d'observation fixe,
- la figure 8 est un extrait d'un film d'acquisition par translation des profils de microlentilles, acquise à 2° d'angle par rapport à la réflexion spéculaire,
- la figure 9 représente une comparaison entre le volume des micro-lentilles mesuré par AFM et celui mesuré par le procédé selon l'invention,

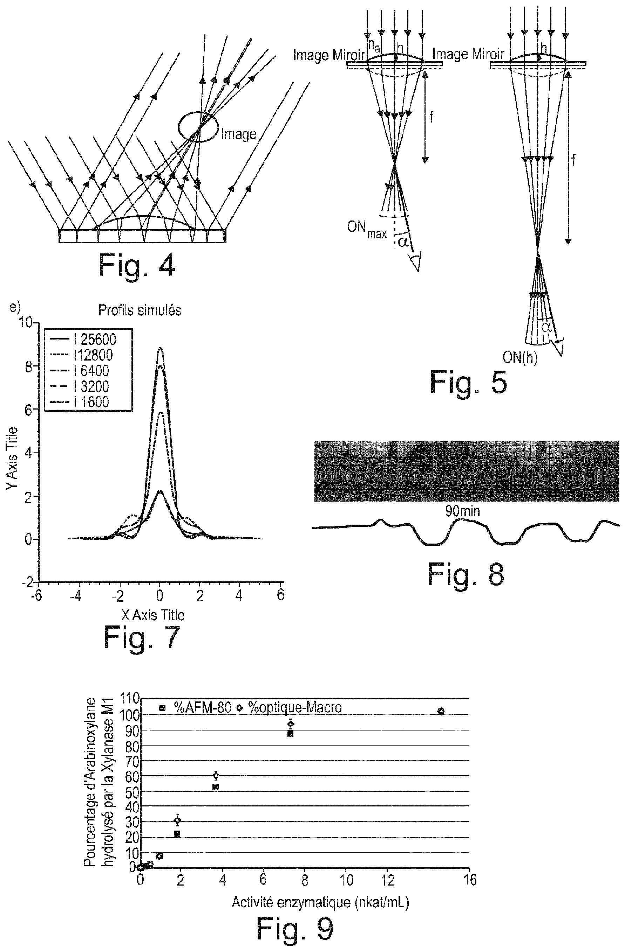

- la figure 9 illustre l'effet de la solution enzymatique sur les micro-dépôts,

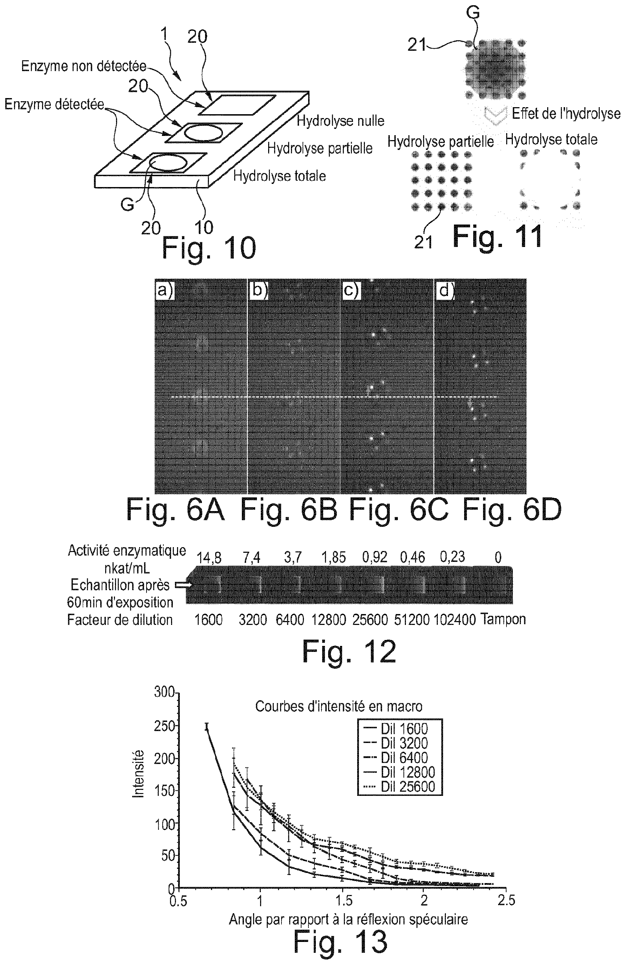

- les figures 10 et 11 illustrent l'effet de la dégradation enzymatique sur des microlentilles,
- la figure 12 est une comparaison de l'activité enzymatique selon différents degrés de dilution, obtenue à partir d'un dispositif selon l'invention, et
- la figure 13 illustre l'intensité de la réflexion des micro-dépôts à différents angles pour différentes dilutions de la solution enzymatique.

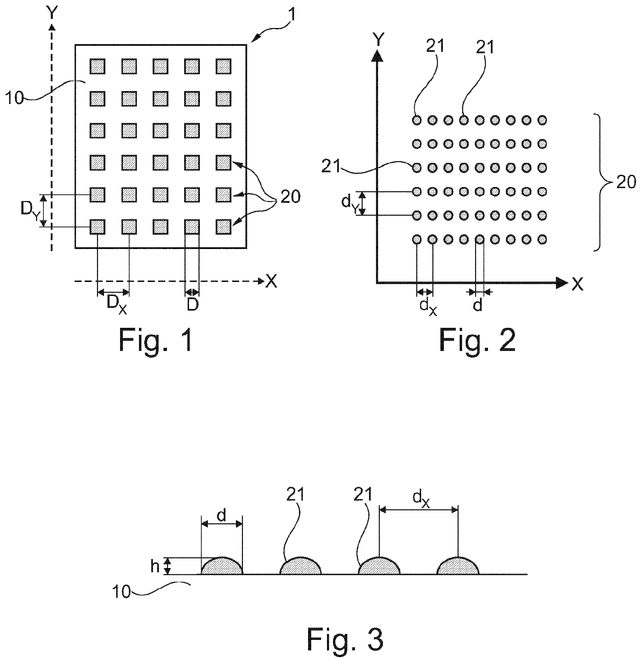

On a représenté à la figure 1 un exemple de dispositif 1 selon l'invention, comportant un support 10 qui est par exemple une plaque de silicium rendue hydrophobe, sur lequel sont formés des macro-motifs 20 sous la forme par exemple de carrés disposés avec des espacements respectifs D_{X} et D_{Y} dans des directions X et Y, correspondant à ceux des puits d'une plaque à puits conventionnelle.

Le côté D d'un macro-motif 20 fait par exemple entre 3 et 5 millimètres et les valeurs D_{X} et D_{Y} sont par exemple comprises entre 3 et 5 mm également.

Chaque macro-motif 20 qui présente une taille millimétrique est formé d'une trame de micro-dépôts 21, ceux-ci étant suffisamment rapprochés pour donner à l'oeil nu à chaque macro-motif 20 un aspect uniforme. Par exemple, l'écartement dₓ dans la direction X et celui d_{y} dans la direction Y, sont compris entre 50 et 340 micromètres, voire 50 et 100 micromètres, le diamètre d d'un micro-dépôt 21 étant par exemple compris entre 10 et 100 micromètres et sa hauteur h (qui correspond à l'épaisseur) entre 100 et 1500 nanomètres

Dans l'exemple considéré, les micro-dépôts 21 sont déposés par impression jet d'encre avec un espacement régulier. L'encre présente une formulation adaptée à la formation d'une surface extérieure convexe.

De préférence, les micro-dépôts 21 permettent, du fait de la différence d'indice de réfraction avec le support 10, ainsi qu'au caractère réfléchissant du support 10, la formation de franges d'interférences telles que des anneaux de Newton. Ces figures d'interférences peuvent être observées au microscope en éclairant le dispositif 1 avec de la lumière blanche.

Le dispositif 1 peut être utilisé en déposant sur chaque macro-motif 20 une goutte G d'un composé à tester, comme illustré aux figures 10 et 11. Selon qu'une réaction de dégradation du polymère par hydrolyse se produit ou non, le changement d'aspect du macro-motif 20 peut être observé à l'oeil nu.

On a représenté sur la figure 11 en haut au milieu la situation où il n'y a pas de réaction entre l'enzyme et le polymère, la réaction d'hydrolyse étant nulle, et en bas à gauche le cas où l'hydrolyse est partielle, voire totale en bas à droite, ce qui permet la détection de l'enzyme.

Cette approche qualitative peut être complétée par une approche quantitative par examen à l'aide d'un système optique qui permet de visualiser l'activité de dégradation d'un ou plusieurs produit(s) chimique(s) sur les dépôts de polymère.

Ce système est composé par exemple d'une source lumineuse éclairant à angle fixe la surface du dispositif et d'un capteur optique (caméra ou autre) collectant la lumière réfléchie par les micro-dépots agissant comme des microlentilles.

Le capteur permet d'extraire des données quantitatives sur la vitesse de dégradation des dépôts grâce à l'impact de la dégradation sur la réponse optique des microlentilles. Une simple lecture de l'intensité lumineuse visible dans les motifs dégradés suffit alors pour quantifier la perte de matière. En effet, lors de la dégradation des lentilles, comme illustré aux figures 4 et 5, leur réduction en diamètre et en épaisseur change leur distance focale et par conséquent leur ouverture numérique. Ainsi, l'évolution de la quantité de lumière reçue à un angle donné est fonction de cette dégradation.

On a représenté sur les figures 6A à 6D des images de micro lentilles selon l'invention, éclairées par une source lumineuse à quatre LEDs.

La figure 6A correspond à une mise au point sur le support, montrant la position des micro-dépôts.

La figure 6B correspond à une mise au point à +/- 100 µm de la surface, mettant en évidence la formation d'une image de la source.

Les figures 6C et 6D illustrent le déplacement de l'image de la source lors du déplacement latéral de la source lumineuse, confirmant l'effet de lentille.

Pour l'acquisition des taux de dégradation du polymère des micro lentilles, deux méthodes sont possibles :
- à angle fixe : la source lumineuse et la caméra sont positionnées de façon statique de part et d'autre du substrat et celui-ci défile par un mouvement de translation. On extrait pour chaque motif le profil d'intensité produit par la dégradation de celui-ci par l'enzyme. Des exemples de profil d'intensité sont donnés à la figure 8, acquis à 2° d'angle par rapport à la réflexion spéculaire.

La figure 7 donne un résultat de simulation montrant la relation entre la dégradation et l'intensité à angle fixe.
- à angle variable : la position d'intérêt est éclairée par une source et la caméra tourne autour de l'échantillon sur un axe. La figure 13 donne l'intensité de la réflexion à différents angles pour différentes dilutions de solution enzymatique.

En extrayant les données obtenues par la seconde méthode à un angle spécifique, on obtient un ensemble de données équivalent à la première méthode.

### EXEMPLES

### Exemple 1

### Première étape : Préparation de l'encre

Une première formulation d'encre aqueuse apte à l'impression par jet d'encre a été préparée selon les spécificités ci-après, où le polymère utilisé est l'arabinoxylane :

| **Encre mono-polymère (formulation 1)** | **Variante 1** | **Variante 2** |
|---|---|---|
| Arabinoxylane Mégazyme-wheat Arabinoxylan - Medium viscosity 22cSt - 323 Kd molecular weight | 5 mg/mL | 3 mg/mL |
| Mélamine formaldéhyde (résine) | 0,025 mg/mL | 0,015 mg/mL |
| ^{®}Resimene AQ-7551 commercialisé par INEOS Melamines | | |
| DMSO CAS 67-68-5 | 10% vol | 10% vol |
| Chlorohydrate de Poly(allylamine) | 0,004 mg/mL | 0,004 mg/mL |
| PAH commercialisé par Polyscience, Mw-120000- 200000 g/mol | | |
| Acide Chlorhydrique HCl | 0,01mM | 0,01mM |

La viscosité de l'encre obtenue selon la variante 1 de la formulation 1 est de 15 mPa.s (à la température d'éjection 20-30°C) et la tension de surface est de l'ordre de 50 Nm (à la température d'éjection).

Dans le cas de la variante 1 de la formulation 1, le ratio polymère/résine est de 200 (5/0,025).

Dans le cas de la variante 2 de la formulation 1, le ratio polymère/résine est également de 200 (3/0,015).

Ci-dessous, une seconde formulation où le polymère utilisé est le bétaglucan, et une troisième formulation où les polymères utilisés sont l'arabinoxylane et le bétaglucan (encre avec mélange de polymères 50%/50%), sont proposées à titre d'exemplification de l'invention :

| **Encre mono-polymère (formulation 2)** | **Variante 1** | **Variante 2** |
|---|---|---|
| Bétaglucan | 7 mg/mL | 3 mg/mL |
| Mélamine formaldéhyde (résine) | 0,025 mg/mL | 0,015 mg/mL |
| ^{®}Resimene AQ-7551 commercialisé par INEOS Melamines | | |
| DMSO CAS 67-68-5 | 10% vol | 10% vol |
| Chlorohydrate de Poly(allylamine) | 0,004 mg/mL | 0,004 mg/mL |
| PAH commercialisé par Polyscience, Mw-120000- 200000 g/mol | | |
| Acide Chlorhydrique HCl | 0,01mM | 0,01mM |

Dans le cas de la variante 1 de la formulation 2, le ratio polymère/résine est de 280 (7/0,025).

Dans le cas de la variante 2 de la formulation 2, le ratio polymère/résine est également de 200 (3/0,015).

| **Encre multi-polymères (formulation 3)** | **Variante 1** | **Variante 2** |
|---|---|---|
| Arabinoxylane Mégazyme-wheat Arabinoxylan - Medium viscosity 22cSt - 323 Kd molecular weight | 2,5 mg/mL | 1,5 mg/mL |
| Bétaglucan | 2,5 mg/mL | 1,5 mg/mL |
| Mélamine formaldéhyde (résine) | 0,025 mg/mL | 0,015 mg/mL |
| ^{®}Resimene AQ-7551 commercialisé par INEOS Melamines | | |
| DMSO CAS 67-68-5 | 10% vol | 10% vol |
| Chlorohydrate de Poly(allylamine) | 0,004 mg/mL | 0,004 mg/mL |
| PAH commercialisé par Polyscience, Mw-120000- 200000 g/mol | | |
| Acide Chlorhydrique HCl | 0,01mM | 0,01mM |

Dans le cas de la variante 1 de la formulation 3, le ratio polymères/résine est de 200 ((2,5+2,5)/0,025).

Dans le cas de la variante 2 de la formulation 3, le ratio polymères/résine est également de 200 ((1,5+1,5)/0,015).

### Deuxième étape : Modification du support

Un wafer de silicium est utilisé comme support.

Afin d'obtenir des dépôts de forme convexe et éviter l'étalement des gouttelettes, la face supérieure du wafer est traitée par C₄F₈ pour être rendue hydrophobe.

### Troisième étape : Impression par jet d'encre

L'encre préalablement préparée selon la variante 1 de la formulation 1 a été chargée dans une imprimante à jet d'encre et des points d'encre sont imprimés sur le support, selon des points de trame.

Les paramètres d'impression sont :
- température d'éjection de l'encre : température ambiante (20°C-30°C),
- volume d'éjection des gouttelettes (par micro-dépôt) : 65 pL +/- 5 pL,
- épaisseur de micro-dépôt : inférieure à 1 µm,
- vitesse d'éjection des gouttelettes : 5 à 6 m/s, et
- macro-motif : matrice carrée de 4 cm de côté avec espacement entre deux micro-dépôts au sein du macro-motif égal à 70 µm.

### Quatrième étape : Réticulation thermique des dépôts d'encre

Le support sur lequel les points d'encre liquide ont été déposés est placé à 130°C pendant 10 à 180 minutes jusqu'à réticulation complète de la résine et solidification des micro-dépôts.

### Mise en œuvre du dispositif de détection

Le dispositif ainsi fabriqué a été mis en oeuvre pour étudier la cinétique de dégradation du polymère présent dans les dépôts, à savoir l'arabinoxylane, par des solutions enzymatiques de xylanase commerciale présentant chacune un niveau d'activité différent.

Tout d'abord, le dispositif a été immergé dans de l'eau à 37°C pendant 2 heures, puis immergé dans une solution aqueuse de BSA (albumine de sérum bovin) à 0,25 g/L à 37°C pendant 1 heure, afin de passiver la surface entre les dépôts de polymère, ce qui améliore la sensibilité des mesures.

Dans cet exemple, la protéine employée en tant qu'agent de passivation est la BSA. Toutefois, l'invention n'est pas limitée à ce cas particulier.

Par exemple, on peut utiliser comme agent de passivation des protéines autres que la BSA, telles que l'ovalbumine ou le lysozyme.

La gamme de concentration en agent de passivation dans le bain dans lequel le dispositif est immergé est préférentiellement comprise entre 0,1g/L et 1g/L.

Des solutions de xylanase de concentration différentes ont été réalisées, présentant des activités enzymatiques allant de 0,23 nkat/mL (dilution à 102 400 - test n°2) jusqu'à 14,8 nkat/mL (dilution à 1 600 - test n°8).

Une goutte de 7 µL de chacune ces solutions est déposée sur un macro-motif correspondant. À titre de témoin, une goutte de tampon acétate est déposée sur un autre motif.

Après 60 minutes d'incubation à température ambiante, rinçage à l'eau et séchage du dispositif, des résultats différents pour chaque concentration ont été observées à l'oeil nu (cf. Tableau ci-dessous).

| N° test | Activité enzymatique (nkat/mL) | Facteur de dilution (par rapport à la solution commerciale de xylanase) | Résultat observé (à l'œil nu) |
|---|---|---|---|
| 1 (témoin) | 0 | - | - |
| 2 | 0,23 | 102 400 | - |
| 3 | 0,46 | 51 200 | - |
| 4 | 0,92 | 25 600 | + |
| 5 | 1,85 | 12 800 | ++ |
| 6 | 3,7 | 6 400 | +++ |
| 7 | 7,4 | 3 200 | +++ |
| 8 | 14,8 | 1 600 | +++ |

Ce test a permis d'estimer un seuil de sensibilité approximativement situé à 0,92 nkat/mL (soit 6,4.10⁻³ nkat).

La figure 9 montre la bonne corrélation entre des mesures du volume des microlentilles effectuées par AFM et des mesures selon l'invention.

### Exemple 2

La figure 12 représente une photographie d'un ensemble de macro-motifs selon l'invention ayant été mis en contact avec différentes concentrations de xylanase (14,8 - 0,23 nkat/mL) durant 60 minutes après hydratation par immersion dans de l'eau pendant 2 heures à 37°C suivie d'une passivation pendant 1 heure dans une solution de BSA à 0,25 g/L à 37°C.

Cette photographie fait apparaître la limite de détection à l'oeil nu pour un facteur de dilution de 25600.

### Liste des documents cités

[1] Huiyan Li et al., « Hydrogel droplet microarrays with trapped antibody-functionalized beads for multiplexed protein analysis », Lab on a Chip, 2011, 11, 528-534
[2] Christiane L. Salgado et al., « Combinatorial cell-3D biomaterials cytocompatibility screening for tissue engineering using bioinspired superhydrophobic substrates », Intégrative Biology, 2012, 4, 318-327
[3] Shouichi Sakakihara et al, « A single-molécule enzymatic assay in a directly accessible femtoliter droplet array », LAB ON A CHIP, vol. 11, no. 24, 2010, page 3355

## Revendications

1. Procédé de détection de la sensibilité d'un ou plusieurs polymères et/ou d'un ou plusieurs mélanges de polymères à un composé, comportant les étapes consistant à :
- exposer au moins un micro-dépôt solide (21) de forme lenticulaire, comportant le ou les polymères et/ou le ou les mélanges de polymères, au composé,
- détecter en éclairant la surface de ce micro-dépôt, un changement de la distribution spatiale de l'intensité de la lumière réfléchie ou transmise par ce micro-dépôt, lié à une variation des dimensions et/ou de l'indice de réfraction de ce micro-dépôt, sous l'effet d'une interaction entre le ou les polymères et/ou le ou les mélanges de polymères et le composé.

2. Procédé selon la revendication 1, plusieurs micro-dépôts étant exposés au composé et la détection du changement de la distribution spatiale de l'intensité lumineuse s'effectuant par l'observation de ces micro-dépôts, la détection du changement de la distribution spatiale étant notamment observée avec ou sans grossissement.

3. Procédé selon la revendication 1, le ou les micro-dépôts étant éclairés par une source ponctuelle ou linéaire, mieux linéaire.

4. Procédé selon l'une quelconque des revendications précédentes, l'observation de la distribution spatiale de l'intensité de la lumière s'effectuant au voisinage de la réflexion spéculaire, notamment dans une plage 0°-2,5° à partir de la réflexion spéculaire, et/ou l'observation du changement de la distribution spatiale s'effectuant avec un capteur matriciel.

5. Procédé selon l'une quelconque des revendications précédentes, dans lequel une information concernant la variation du volume du micro-dépôt est générée à partir du changement observé, par comparaison à des données de référence.

6. Procédé selon l'une quelconque des revendications 1 à 5, l'épaisseur des micro-dépôts étant comprise entre 100 et 5000 nm, et/ou la plus grande dimension des micro-dépôts étant comprise entre 10 et 100 microns, et/ou l'épaisseur de chaque micro-dépôt passant par un seul maximum, et/ou chaque micro-dépôt (21) ayant une surface extérieure convexe.

7. Procédé selon l'une quelconque des revendications précédentes, les micro-dépôts étant disposés sous forme de points de trame, et/ou les micro-dépôts étant disposés selon un réseau périodique et éclairés avec une lumière cohérente, et l'on observe la distribution spatiale de la lumière dans une figure de diffraction produite par le réseau, afin de mesurer la dégradation du ou des polymères, et/ou les micro-dépôts (21) étant disposés selon des macro-motifs (20) distants les uns des autres, de préférence disposés comme les puits d'une grille multi-puits pour dépôt robotisé, et/ou les micro-dépôts étant situés sur un support (10) réfléchissant et/ou hydrophobe.

8. Procédé selon l'une quelconque des revendications précédentes, le composé étant une enzyme, l'interaction entre le composé et le ou les polymères étant de préférence une dégradation du ou des polymères par le composé, et/ou le ou les polymères étant des bio-polymères, notamment choisi parmi dans le groupe constitué par les oligosaccharides et polysaccharides tels que la cellulose, le xylane, la pectine, le chitosane, la chitine, le xyloglucane, le bétaglucan, et l'arabinoxylane ; les peptides et les protéines tels que l'albumine de sérum bovin ou humain et la gluténine ; les acides nucléiques tels que les acides désoxyribonucléiques et les acides ribonucléiques ; la cutine, la subérine et la lignine.

9. Dispositif pour la mise en oeuvre du procédé selon l'une quelconque des revendications précédentes, comportant :
- un support (10),
- une pluralité de micro-dépôts (21) solides individualisés portés par le support, de forme lenticulaire, chaque micro-dépôt comportant au moins un polymère sensible à un composé.

10. Dispositif selon la revendication 9, la plus grande dimension des micro-dépôts étant comprise entre 10 et 100 microns, et/ou l'épaisseur (h) de chaque micro-dépôt passant par un seul maximum, et/ou chaque micro-dépôt (21) ayant une surface extérieure convexe, et/ou les micro-dépôts étant disposés sous forme de points de trame, et/ou les micro-dépôts étant disposés selon des macro-motifs (20) distants les uns des autres, de préférence disposés comme les puits d'une grille multi-puits pour dépôt robotisé, et/ou les micro-dépôts (21) étant disposés de façon suffisamment proche pour que l'effet d'interaction entre le ou les polymères et le composé soit visible à l'oeil nu sous forme d'un contraste d'intensité, et/ou le support étant réfléchissant et/ou hydrophobe, notamment traité par un matériau fluoré.

11. Ensemble comportant un dispositif tel que défini dans la revendication 9 ou 10 et un composé interagissant avec le ou les polymères pour conduire à une variation des dimensions et/ou de l'indice de réfraction dudit dépôt, le composé étant notamment une enzyme propre à dégrader le ou les polymères.

12. Procédé de fabrication d'un dispositif tel que défini dans la revendication 9 ou 10, comportant le dépôt par impression sur le support de points d'une encre contenant le ou les polymères, un liant et un co-solvant organique choisi pour permettre la formation des micro-dépôts de forme convexe lors du séchage de l'encre sur le support.

13. Procédé de détection d'un changement d'état d'un ou plusieurs polymères sous l'action d'un composé auquel le ou les polymères sont sensibles, comportant les étapes consistant à :
- exposer au moins un micro-dépôt solide de forme lenticulaire, comportant le ou les polymères, à des conditions provoquant un changement d'état au moins partiel,
- détecter en éclairant la surface de ce micro-dépôt, un changement de la distribution spatiale de l'intensité de la lumière réfléchie ou transmise par ce micro-dépôt, lié à une variation des dimensions et/ou de l'indice de réfraction de ce micro-dépôt sous l'effet de ce changement d'état.

14. Procédé de détection de la sensibilité d'un ou plusieurs polymères à un composé, comportant les étapes consistant à :
- exposer un arrangement périodique-de micro-dépôts solides de forme lenticulaire, comportant le ou les polymères, au composé,
- détecter en exposant la surface de cet arrangement périodique à une lumière adaptée, notamment cohérente, un changement de la distribution spatiale de l'intensité de la lumière diffractée par ce réseau, lié à une variation des dimensions et/ou de l'indice de réfraction des micro-dépôts de cet arrangement sous l'effet d'une interaction entre le ou les polymères et le composé.

## Patentansprüche

1. Verfahren zur Detektion der Empfindlichkeit eines oder mehrerer Polymere und/oder einer oder mehrerer Polymermischungen gegenüber einer Verbindung, wobei es die folgenden Schritte aufweist:
- Einwirkenlassen der Verbindung auf mindestens eine linsenförmige feststoffliche Mikroabscheidung (21), welche das oder die Polymere und/oder die Polymermischung(en) aufweist,
- Detektieren, indem die Oberfläche dieser Mikroabscheidung beleuchtet wird, einer Veränderung der räumlichen Verteilung der Intensität des Lichts, welches von dieser Mikroabscheidung reflektiert oder hindurchgelassen wird, wobei diese mit einer Variation der Abmessungen und/oder des Brechungsindex der Mikroabscheidung durch die Auswirkung einer Wechselwirkung zwischen dem oder den Polymeren und/oder der und den Polymermischungen und der Verbindung in Zusammenhang steht.

2. Verfahren nach Anspruch 1, wobei die Verbindung auf mehrere Mikroabscheidungen einwirkt und die Detektion der Veränderung der räumlichen Verteilung der Lichtintensität erfolgt, indem diese Mikroabscheidungen beobachtet werden, wobei die Detektion der Veränderung der räumlichen Verteilung insbesondere mit oder ohne Vergrößerung beobachtet wird.

3. Verfahren nach Anspruch 1, wobei die Mikroabscheidung(en) von einer punktartigen oder linearen, besser linearen, Lichtquelle beleuchtet werden.

4. Verfahren nach einem beliebigen der vorhergehenden Ansprüche, wobei die Beobachtung der räumlichen Verteilung der Intensität des Lichts in der Nähe der Spiegelreflexion erfolgt, insbesondere in einem Bereich von 0° bis 2,5° ausgehend von der Spiegelreflexion und/oder die Beobachtung der Veränderung der räumlichen Verteilung mit einem matrixartigen Sensor erfolgt.

5. Verfahren nach einem beliebigen der vorhergehenden Ansprüche, wobei eine Information, welche die Variation des Volumens der Mikroabscheidung betrifft, ausgehend von der beobachteten Veränderung erzeugt wird, durch Vergleich mit Referenzdaten.

6. Verfahren nach einem beliebigen der Ansprüche 1 bis 5, wobei die Dicke der Mikroabscheidungen im Bereich von 100 bis 5000 nm liegt und/oder die größte Abmessung der Mikroabscheidungen im Bereich von 10 bis 100 Mikrometern liegt und/oder die Dicke jeder der Mikroabscheidungen ein einziges Maximum durchläuft und/oder jede der Mikroabscheidungen eine konvexe Außenfläche hat.

7. Verfahren nach einem beliebigen der vorhergehenden Ansprüche, wobei die Mikroabscheidungen in Form von Rasterpunkten angeordnet sind und/oder die Mikroabscheidungen gemäß einem wiederkehrenden Gittermuster angeordnet sind und mit einem kohärenten Licht beleuchtet werden, wobei die räumliche Verteilung des Lichts in einer Beugungsfigur beobachtet wird, wie sie von dem Gitter erzeugt wird, um den Abbau des oder der Polymere zu messen, und/oder die Mikroabscheidungen (21) gemäß voneinander beabstandeten Makromotiven (20) angeordnet sind, wobei diese vorzugsweise wie die Vertiefungen eines Rasters mit mehreren Vertiefungen für ein robotisiertes Aufbringen angeordnet sind, und/oder die Mikroabscheidungen sich auf einem reflektierenden und/oder hydrophoben Träger (10) befinden.

8. Verfahren gemäß einem beliebigen der vorhergehenden Ansprüche, wobei die Verbindung ein Enzym ist, wobei es sich bei der Wechselwirkung zwischen der Verbindung und dem oder den Polymeren vorzugsweise um einen Abbau des oder der Polymere durch die Verbindung handelt, und/oder es sich bei dem oder den Polymeren um Biopolymere handelt, die insbesondere aus der Gesamtheit ausgewählt sind, welche aus den Oligosacchariden und Polysacchariden wie etwa Cellulose, Xylan, Pektin, Chitosan, Chitin, Xyloglucan, Betaglucan und Arabinoxylan; den Peptiden und den Proteinen wie etwa Serumalbumin vom Rind oder vom Menschen sowie Glutenin; den Nukleinsäuren wie etwa den Desoxyribonukleinsäuren und den Ribonukleinsäuren; aus Cutin, Suberin und Lignin besteht.

9. Vorrichtung zur Durchführung des Verfahrens gemäß einem beliebigen der vorhergehenden Ansprüche, wobei sie Folgendes aufweist:
- einen Träger (10),
- eine Mehrzahl an einzelnen feststofflichen Mikroabscheidungen (21), die sich auf dem Träger befinden, wobei sie linsenförmig sind und jede der Mikroabscheidungen mindestens ein Polymer aufweist, welche gegenüber einer Verbindung empfindlich ist.

10. Vorrichtung nach Anspruch 9, wobei die größte Abmessung der Mikroabscheidungen im Bereich von 10 bis 100 Mikrometern liegt und/oder die Dicke (h) jeder der Mikroabscheidungen ein einziges Maximum durchläuft und/oder jede der Mikroabscheidungen (21) eine konvexe Außenfläche hat und/oder die Mikroabscheidungen in Form von Rasterpunkten angeordnet sind und/oder die Mikroabscheidungen gemäß voneinander beabstandeten Makromotiven (20) angeordnet sind, die vorzugsweise wie die Vertiefungen eines Rasters mit mehreren Vertiefungen für ein robotisiertes Aufbringen angeordnet sind, und/oder die Mikroabscheidungen (21) in ausreichender Nähe angeordnet sind, dass die Auswirkungen der Wechselwirkung zwischen dem oder den Polymeren und der Verbindung mit bloßem Auge als Intensitätskontrast wahrnehmbar sind, und/oder der Träger reflektierend und/oder hydrophob ist, wobei er vorzugsweise mit einem fluorhaltigen Material behandelt ist.

11. System, das eine Vorrichtung gemäß der Begriffsbestimmung in Anspruch 9 oder 10 sowie eine Verbindung aufweist, welche mit dem oder den Polymeren in Wechselwirkung tritt, um eine Variation der Abmessungen und/oder des Brechungsindex der Abscheidung zu bewirken, wobei es sich bei der Verbindung insbesondere um ein Enzym handelt, welches dazu geeignet ist, das oder die Polymere abzubauen.

12. Verfahren zur Herstellung einer Vorrichtung gemäß der Begriffsbestimmung in Anspruch 9 oder 10, wobei es das Abscheiden auf dem Träger, mittels eines Druckverfahrens, von Punkten einer Druckfarbe aufweist, welche das oder die Polymere, ein Bindemittel und ein organisches Hilfslösungsmittel enthält, welches derart gewählt ist, dass sich Mikroabscheidungen von konvexer Form bilden können, wenn die Druckfarbe auf dem Träger trocknet.

13. Verfahren zur Detektion einer Zustandsänderung eines oder mehrerer Polymere unter Einwirkung einer Verbindung, gegenüber welcher das oder die Polymere empfindlich sind, wobei es die folgenden Schritte aufweist:
- Einwirkenlassen von Bedingungen, die eine zumindest teilweise Zustandsänderung bewirken, auf mindestens eine linsenförmige feststoffliche Mikroabscheidung, welche das oder die Polymere aufweist,
- Detektieren, indem die Oberfläche dieser Mikroabscheidung beleuchtet wird, einer Veränderung der räumlichen Verteilung der Intensität des Lichts, welches von dieser Mikroabscheidung reflektiert oder hindurchgelassen wird, wobei diese mit einer Variation der Abmessungen und/oder des Brechungsindex der Mikroabscheidung durch die Auswirkung dieser Zustandsänderung in Zusammenhang steht.

14. Verfahren zur Detektion der Empfindlichkeit eines oder mehrerer Polymere gegenüber einer Verbindung, wobei es die folgenden Schritte aufweist:
- Einwirkenlassen der Verbindung auf eine wiederkehrende Anordnung von linsenförmigen feststofflichen Mikroabscheidungen,
- Detektieren, indem ein geeignetes Licht, insbesondere kohärenter Art, auf die Oberfläche dieser wiederkehrenden Anordnung einwirken gelassen wird, einer Veränderung der räumlichen Verteilung der Intensität des Lichts, welches von diesem Gitter gebeugt wird, wobei diese mit einer Variation der Abmessungen und/oder des Brechungsindex der Mikroabscheidungen dieser Anordnung durch die Auswirkung einer Wechselwirkung zwischen dem oder den Polymeren und der Verbindung in Zusammenhang steht.

## Claims

1. Process for detecting the sensitivity of one or more polymers and/or of one or more mixtures of polymers to a compound, including the steps consisting in:
- exposing at least one lens-shaped solid micro-deposit (21), including the polymer(s) and/or the mixture(s) of polymers, to the compound,
- detecting, by illuminating the surface of this micro-deposit, a change in the spatial distribution of the intensity of the light reflected or transmitted by this micro-deposit, linked to a variation in the dimensions and/or refractive index of this micro-deposit, under the effect of an interaction between the polymer(s) and/or the mixture(s) of polymers and the compound.

2. Process according to Claim 1, in which several micro-deposits are exposed to the compound and the detection of the change in spatial distribution of the light intensity is performed by observation of these micro-deposits, the detection of the change in spatial distribution notably being observed with or without magnification.

3. Process according to Claim 1, in which the micro-deposit(s) are illuminated with a point or linear source, better still a linear source.

4. Process according to any one of the preceding claims, in which the observation of the spatial distribution of the light intensity is performed in the region of the specular reflection, notably in a range 0°-2.5° from the specular reflection, and/or the observation of the change in spatial distribution is performed with a matrix sensor.

5. Process according to any one of the preceding claims, in which information regarding the variation in the volume of the micro-deposit is generated from the change observed, by comparison with reference data.

6. Process according to any one of Claims 1 to 5, in which the thickness of the micro-deposits is between 100 and 5000 nm, and/or the largest dimension of the micro-deposits is between 10 and 100 microns, and/or the thickness of each micro-deposit passes through a single maximum, and/or each micro-deposit (21) has a convex outer surface.

7. Process according to any one of the preceding claims, in which the micro-deposits are arranged in the form of raster dots, and/or micro-deposits are arranged in a periodic network and illuminated with coherent light, and the spatial distribution of the light is observed in a diffraction pattern produced by the network, in order to measure the degradation of the polymer(s), and/or the micro-deposits (21) are arranged in macro-patterns (20) separated from each other, preferably arranged like the wells of a multi-well grid for automated deposition, and/or the micro-deposits are located on a reflective and/or hydrophobic support (10).

8. Process according to any one of the preceding claims, in which the compound is an enzyme, the interaction between the compound and the polymer(s) is preferably a degradation of the polymer(s) by the compound, and/or the polymer(s) are biopolymers, notably chosen from the group consisting of oligosaccharides and polysaccharides such as cellulose, xylan, pectin, chitosan, chitin, xyloglucan, beta-glucan and arabinoxylan; peptides and proteins such as bovine or human serum albumin and glutenin; nucleic acids such as deoxyribonucleic acids and ribonucleic acids; cutin, suberin and lignin.

9. Device for performing the process according to any one of the preceding claims, including:
- a support (10),
- a plurality of individualized solid lens-shaped micro-deposits (21) borne by the support, each micro-deposit including at least one polymer that is sensitive to a compound.

10. Device according to Claim 9, in which the largest dimension of the micro-deposits is between 10 and 100 microns, and/or the thickness (h) of each micro-deposit passes through a single maximum, and/or each micro-deposit (21) has a convex outer surface, and/or the micro-deposits are arranged in the form of raster dots, and/or the micro-deposits being arranged in macro-patterns (20) separated from each other, preferably arranged like the wells of a multi-well grid for automated deposition, and/or the micro-deposits (21) are positioned closely enough so that the interaction effect between the polymer(s) and the compound is visible to the naked eye in the form of an intensity contrast, and/or the support is reflective and/or hydrophobic, notably treated with a fluorinated material.

11. Assembly including a device as defined in Claim 9 or 10 and a compound interacting with the polymer(s) to lead to a variation in the dimensions and/or refractive index of said deposit, in which the compound is notably being an enzyme that is capable of degrading the polymer(s).

12. Process for manufacturing a device as defined in Claim 9 or 10, including the deposition by printing onto the support of dots of an ink containing the polymer(s), a binder and an organic cosolvent chosen to allow the formation of convex-shaped micro-deposits during the drying of the ink on the support.

13. Process for detecting a change of state of one or more polymers under the action of a compound to which the polymer(s) are sensitive, including the steps consisting in:
- exposing at least one lens-shaped solid micro-deposit, including the polymer(s), to conditions causing an at least partial change of state,
- detecting, by illuminating the surface of this micro-deposit, a change in the spatial distribution of the intensity of the light reflected or transmitted by this micro-deposit, linked to a variation in the dimensions and/or refractive index of this micro-deposit, under the effect of this change of state.

14. Process for detecting the sensitivity of one or more polymers to a compound, including the steps consisting in:
- exposing a periodic arrangement of lens-shaped solid micro-deposits, including the polymer(s), to the compound,
- detecting, by exposing the surface of this periodic arrangement to a suitable light, notably coherent light, a change in the spatial distribution of the intensity of the light diffracted by this network, linked to a variation in the dimensions and/or refractive index of the micro-deposits of this arrangement under the effect of an interaction between the polymer(s) and the compound.
